Europäisches Patentamt

European Patent Office

Office européen des brevets

(19)

(11) EP 0 671 958 B1

(12) **EUROPEAN PATENT SPECIFICATION**

(45) Date of publication and mention
of the grant of the patent:
**18.07.2001 Bulletin 2001/29**

(21) Application number: **94924858.7**

(22) Date of filing: **09.08.1994**

(51) Int Cl.[7]: **A61M 1/36**

(86) International application number:
**PCT/EP94/02640**

(87) International publication number:
**WO 95/04560 (16.02.1995 Gazette 1995/08)**

(54) **APPARATUS FOR THE TREATMENT OF TUMORS BY SELECTIVE PROTEIN DEPLETION**

GERÄT ZUM BEHANDELN VON TUMOREN MITTELS SELEKTIVER PROTEINVERRINGERUNG

DISPOSITIF POUR TRAITEMENT DE TUMEURS PAR EPUISEMENT PROTEIQUE SELECTIF

(84) Designated Contracting States:
**AT BE CH DE DK ES FR GB GR IE IT LI LU MC NL PT SE**

(30) Priority: **10.08.1993 US 104984**

(43) Date of publication of application:
**20.09.1995 Bulletin 1995/38**

(73) Proprietor: **Tepic, Slobodan**
**8006 Zürich (CH)**

(72) Inventor: **Tepic, Slobodan**
**8006 Zürich (CH)**

(74) Representative: **Lusuardi, Werther Giovanni, Dr.**
**Dr. Lusuardi AG,**
**Kreuzbühlstrasse 8**
**8008 Zürich (CH)**

(56) References cited:
**GB-A- 2 020 668          US-A- 4 955 857**

- **BIOTECHNOLOGY AND BIOENGINEERING. INCLUDING: SYMPOSIUM BIOTECHNOLOGY IN ENERGY PRODUCTION AND CONSERVATION, vol.35, 1990, NEW YORK US pages 260 - 267 S.R. REIKEN ET AL. 'THE USE OF A SINGLE-FIBER REACTOR FOR THE ENZYMATIC REMOVAL OF AMINO ACIDS FROM SOLUTIONS.'**
- **ANNALS OF THE NEW YORK ACADEMY OF SCIENCES, vol.613, 1990, NEW YORK, N.Y., US pages 489 - 493 B. TONOMURA ET AL. 'ATP BINDING PLAYS A ROLE IN THE SELECTION OF AMINO ACID SUBSTRATE BY AMINOACYL-tRNA SYNTHETASES.'**

## Description

## FIELD OF THE INVENTION

This invention relates to a treatment of tumors based on a universal difference between normal and tumor cells in the dynamics of growth. This difference in growth dynamics accounts for controllability of the system of mixed populations of normal and tumor cells by means of a common source of nutrients - blood circulation. Using the concentration of one essential nutrient (preferably an essential amino acid) in the blood, tumor cells anywhere in the body can be selectively depleted of essential products (preferably proteins) to the level insufficient for survival, while normal cells continue to live and, those cells which normally do so, proliferate following the treatment.

## BACKGROUND ART

Cancer is the second (after cardio-vascular diseases) leading cause of death in the developed world. An enormous research effort of the last decades has produced dramatic advances in understanding mechanisms of transformation, i.e. of the process by which a normal cell becomes cancerous. The pace of discovery has quickened in the last several years with new tools of molecular biology coming to aid, many of which have actually been developed in the effort to understand cancer. Unfortunately, the treatment of cancers has not seen much improvement, and with several notable exceptions, the five-year survival rate has remained about the same throughout this period of several decades - some 50% overall.

In multicellular organisms, division of an individual cell is an event controlled by the needs of the whole organism. While most cells are capable of dividing, or mitosis, they rarely do so if not stimulated to by the conditions of the tissues they form. If an injury is inflicted, for example, the local, as well as the infiltrating cells, may respond by mitosis and tissue regeneration in order to repair the damage. Once the repair is done, the cells return to their quiet existence without proliferation. In some cases, the division of cells is a rule rather than exception. For example, in the bone marrow, cell proliferation continuously provides for blood cells replenishment. The intestinal lining cells also proliferate continuously in order to make up for the loss of the outermost layers where in the harsh environment cells do not last very long. In a healthy individual the steady state is well controlled by local conditions of blood supply, geometrical intercellular relationships, territorial integrity, as well as by systemic factors such as growth factors production, nutrient availability, and the like. The imbalance between cell proliferation and cell death caused by the loss of normal mitotic cycle controls leads to a tumor or neoplasm. If the growth remains local, the tumor is said to be benign, and a complete surgical resection leads to cure. Some tumors, however, possess mechanisms needed for the spread into, and proliferation in other tissues. Such tumors are characterized as malignant, and are referred to as cancers. The spread involves cell separation from the local tumor mass, entry into the blood or lymphatic circulation, transport to another site, exit into and continued growth at inappropriate sites. Treatment of cancers which have spread to various locations, and have formed the secondary tumors, or metastases, is very difficult - in order to succeed, the attack must be selective. Finding selective strategies is the main topic of clinical cancer research efforts. Indeed, the possibility of discovering a successful cancer treatment must be the main motivation of all research on cancer and related aspects of cell biology.

In general, tumors appear to be monoclonal, i. e. all of the tumor cells have descended from a single progenitor cell. Transformation which has made the progenitor cell cancerous is a slow, multiple stage process, requiring in most known cases a number of specific genetic defects. The genes affected are called oncogenes and the products they encode oncoproteins. The changes in DNA sequence may be produced by chemical carcinogens, ionizing radiation or viral infection, but many other factors play a role in the process. The end effect by which the cell is recognized as tumorous is the apparent lack of proliferation control. To decide whether a cell is transformed or not one can make two functional tests: (1) if the cell divides in suspension, i.e. without "anchorage" ; or (2) if the cell grows into a tumor in a nude mouse (a mouse with no immune system), the cell most likely is transformed. The discovery of the first oncogen inspired a great deal of optimism based on the hope that perhaps only that single defect needed to be somehow corrected to cure cancer. But tens of oncogens (just over one hundred by now) were identified very quickly and it became clear that cancer was what it has been taken for - a multitude of diseases. Nevertheless, they all do lead to very similar manifestations and ultimate common path in the death of the patient, keeping the hope alive that there might be a single cure yet.

As of now, the surgical treatment, whenever possible, is still the most efficient treatment - if the cancer has not spread from its primary site, the complete resection of the tumor leads to cure of the cancer. If surgery is not possible, or the spread of cancer cells has occurred prior to surgery, chemotherapy may kill some types of cancers. Not all types are susceptible, however, and the treatment is in any case a balancing game - killing as much of the cancer without killing the patient. The toxic chemicals used for chemotherapy are specific to different phases of the cell cycle, and only a number of cells will be killed by any single dose - some of them cancerous, some of them normal cells that proliferate continuously (most importantly cells in the bone marrow and intestines). Treatment protocols have been developed over years of experimentation and clinical use aimed at combining different drugs in ways to maximize the chances of cancer elimination. Radiation treatment is another possibility, used mostly in conjunction with surgery. In this case, again, the problem is differentiating sufficiently between the normal and cancerous tissue. Even when the cancer is spatially distinct, the methods of radiation delivery available today are not precise enough. Asynchronous cell proliferation is a major drawback here as well - cells are not equally susceptible to radiation in different parts of the cycle.

Other physical treatment approaches have been tried and have to a great extent remained experimental - local hyperthermia (produced by ultrasound), for example, has been employed as an adjunct to chemotherapy.

Most promising of the new approaches are those based on using either naturally occurring, or engineered, substances that can interfere with cancer growth and spread: Tumor Necrosis Factor has been identified and tested in native and modified forms; Lymphokine Activated Killer cells have been prepared and used in conjunction with interleukine-2; vaccination against melanoma, which appears to have characteristic surface markers, has been under development; "magic bullet" drugs, i.e. cytotoxic drugs targeted by the aid of specific antibodies, show a great promise against cancers that display antigens not found on the normal cells, etc. As the details of transformation fill in, new possibilities will certainly open up. Just over one hundred oncogenes have been identified - the proteins they encode are found at different locations within the cell, and a troubling possibility exists that many cancer cells may not be identified as such by their surface antigens. Entering the cell in order to intervene, while not impossible, is going to be a lot more difficult than to exert the action on the surface. And nothing very efficient has been done even for those types of cancer that do possess strong surface antigens.

The unique approach presented here is based on the most universal of the features of all tumor cells - the property that in fact defines them as tumorous - their propensity to grow and proliferate under conditions where normal cells would not.

## BRIEF DESCRIPTION OF THE DRAWINGS

In the drawings:

Figure 1    Self-enlarging synthesis plant
Figure 2    Numerical solutions to the growth function
Figure 3    A graph of the effect on the cell growth function of the initial condition of the cells prior to treatment using the system of the present invention
Figure 4    Cell cycle representation on the $m_U$ curves
Figure 5    Relative positions of the cell cycle on the $m_U$ curves
Figure 6    A simple, three-tank, controllable system
Figure 7    A basic control strategy to selectively kill tumor cells
Figure 8    Basic blood filtration system
Figure 9    Blood filtration system with the secondary circulation
Figure 10   Enzymes immobilized on direct contact surfaces
Figure 11   Enzymes immobilized behind a molecular sieve
Figure 12   Secondary circulation with enzymes in solution
Figure 13   Secondary circulation with gel-bead-bound enzymes
Figure 14   Two-stage filtration
Figure 15   Multiple stage filtration with a secondary circulation
Figure 16   A complete system for extracorporeal blood treatment

## DETAILED DESCRIPTION OF THE INVENTION

### Growth model

For the benefit of the reader and better understanding of the invention, an original (unpublished) cell growth model is presented first. It successfully describes the known features of cell growth and allows for a rational discussion of the cell cycle. The treatment proposed is based on, and can be best understood with the help of the growth/cycle diagrams which result from the model.

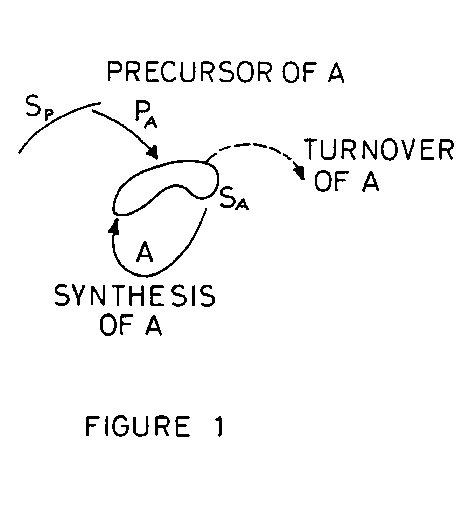

In this model, the product A is synthesized in a synthesis plant. *A region of synthesis of the product $A$ is denoted by $S_A$. In the case of a cell, this region of synthesis would be typically on an internal membrane. The precursor of $A$ is supplied by the source $S_P$, as shown on Figure 1.

Assumptions:

(a) the synthesis plant is a self-enlarging plant so that the synthesis of product $A$ at synthesis region $S_A$ results in an enlargement of synthesis region $S_A$ ;

(b) $A$ undergoes turnover;

(c) precursor $P_A$ is supplied via resistive path - path length is proportional to the square root of the size of $S_A$ (see assumption (d)), i.e. system is uniformly expanding;

(d) $S_A$ is a 2D structure, i.e. mass of $A$ at $S_A$ is proportional to the surface of $S_A$.

Note: Assumption (d) is not of crucial importance - it merely changes an exponent in the final equation. If bio-mass (macro-molecular substances) is concentrated on membranes it would be proportional to $\delta^2$, where $\delta$ is a linear measure of the cell size. If the bio-mass was concentrated in volumes, mass would be proportional to $\delta^3$. In reality **m** is proportional to $\delta^x$, where

2<x<3 (and probably closer to 2).

In the equations of growth variables are defined as follows:

$t$ - time

$m_A$ - mass of $A$ at $S_A$

$m_P$ - mass of precursor $P_A$ at $S_A$

$C_{P0}$ - concentration of precursor $P_A$ at $S_P$, whereby $S_P$ denotes the source of the precursor $P_A$;

$c_{PA}$ - concentration of precursor $P_A$ at $S_A$

$c_{AA}$ - concentration of $A$ at $S_A$

$m_{AS}$ - mass of $A$ synthesized at $S_A$.

Flow of precursor between $S_P$ and $S_A$ and its mass balance at $S_A$ :

$$\frac{dm_P}{dt} = \frac{c_{P0} - c_{PA}}{R} - \frac{dm_{AS}}{dt}, \qquad (1)$$

where $R$ is the total resistance from $S_P$ to $S_A$

Rate of synthesis of $A$ at $S_A$ :

$$\frac{dm_{AS}}{dt} = \alpha \, c_{PA} \, m_A, \qquad (2)$$

where $\alpha$ is a constant,

$c_{PA}$ is the concentration of precursor at $S_A$, and $m_A$ is the mass of $A$ at $S_A$ (size of synthesizing region). We assume that concentration $C_{AA}$ of $A$ at $S_A$ remains constant as $S_A$ grows.

Mass balance of $A$ at $S_A$ :

$$dm_A = dm_{AS} - \gamma m_A \, dt, \qquad (3)$$

where $\gamma$ is the turnover constant.

Assuming for now no accumulation of the precursor at $S_A$, i.e. setting

$$\frac{dm_P}{dt} = 0, \qquad (1)$$

gives:

$$\frac{dm_{AS}}{dt} = \frac{c_{P0} - c_{PA}}{R} \qquad (4)$$

and (2), (4) give:

$$\frac{dm_{AS}}{dt} = \frac{c_{P0}}{R} - \frac{1}{\alpha \, R \, m_A} \frac{dm_{AS}}{dt}, $$

which gives

$$\frac{dm_{AS}}{dt} = \frac{c_{P0} \, \alpha \, m_A}{\alpha \, R \, m_A + 1} \qquad (5)$$

Now (3), (5) give:

$$\frac{dm_A}{dt} = \frac{c_{P0} \, \alpha \, m_A}{\alpha \, R \, m_A + 1} - \gamma m_A \qquad (6)$$

With the assumption (d), i.e. $m_A$ is proportional to $\delta^2$, we can state:

$$R = \rho \sqrt{m_A} \, ,$$

and rewrite (6) as:

$$\frac{dm_A}{dt} = \frac{c_{P0} \, \alpha \, m_A}{\alpha \, \rho \, m_A^{3/2} + 1} - \gamma m_A \qquad (7)$$

Equation (7) is a non-linear differential equation that describes growth of the bio-synthesizing region $S_A$ modelled with assumptions (a) to (d). Parameters in the equation:

$c_{P0}$ - concentration of the precursor at its source $S_P$

$\alpha$ - synthesis constant

$\gamma$ - turnover constant

$\rho$ - resistance for precursor transport.

Equation (7) has no closed form solution. So we can look at some basic properties of the solution, and than solve the equation numerically.

Leaving the subscripts out for simplicity we can rewrite:

$$\frac{dm}{dt} = \frac{c \, \alpha \, m}{\alpha \, \rho \, m^{3/2} + 1} - \gamma m \qquad (8)$$

Firstly, note that

$$\frac{dm}{dt}$$

is a function of $m$, but not of $t$.

Let

$$\frac{dm}{dt} = 0$$

to find extrema:

$$m_{e\,1} = 0, \qquad m_{e2} = [\frac{1}{\rho}(\frac{c}{\gamma}-\frac{1}{\alpha})]^{2/3}$$

For physical relevance $\rho$, $\gamma$, $\alpha > 0$. Several special features of the solution to (8) :

* If $\alpha = 0$, (8) reduces to

$$\frac{dm}{dt} = -\gamma m,$$

which leads to $m = m_0\,e^{-\gamma t}$, i.e. $m$ will exponentially decay from its initial value $m_0$ to 0;

* If $\alpha > 0$ and either $\rho = 0$, or $\gamma = 0$ $m$ will grow without bounds;

* For $\alpha > 0$, $\rho > 0$, and $\gamma > 0$ $m$ will grow (or shrink, depending on $m_0$) asymptotically to $m_e$.

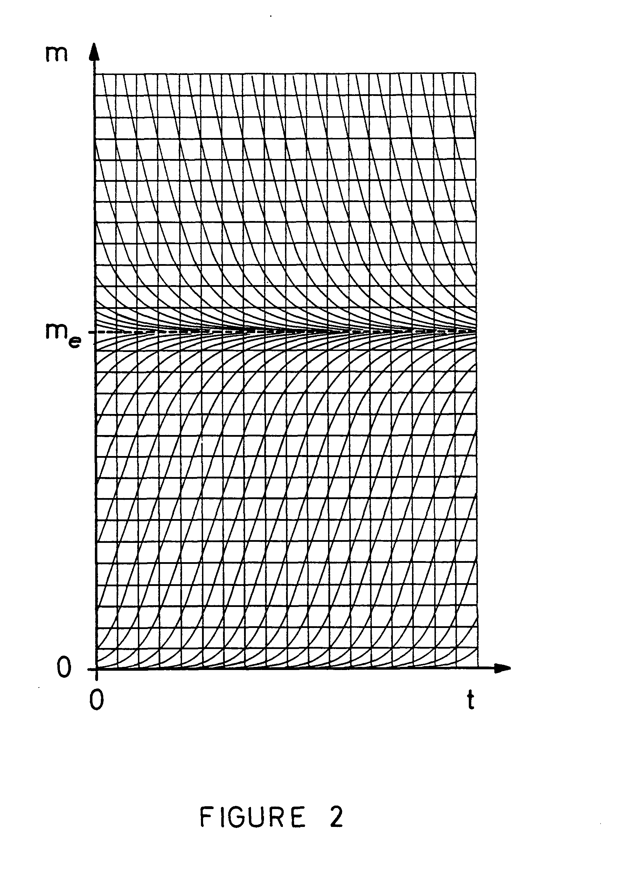

Figure 2 shows solutions to (8) found numerically using Runge-Kutta method. Note the influence of the initial condition $m(0) = m_0$ on the solution:

* If $m_0 = 0$, then $m=0$;
* If $0<m_0<m_e$, $m$ asymptotically grows to

$$m_{e2} = [\frac{1}{\rho}(\frac{c}{\gamma}-\frac{1}{\alpha})]^{2/3};$$

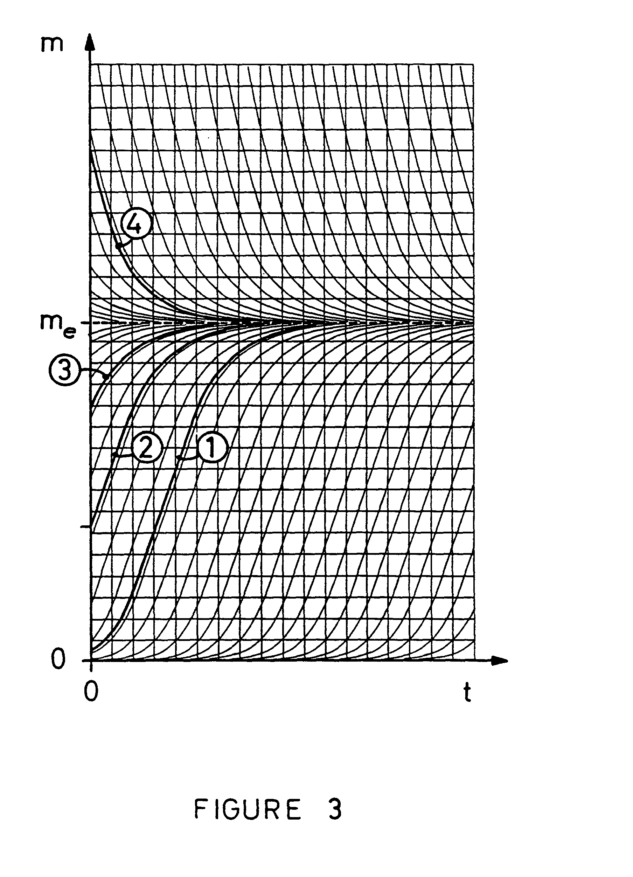

* If $m_0 = m_e$, then $m = m_0$;
* If $m_0 > m_e$, $m$ asymptotically shrinks to $m_e$. (curve 4, Figure 3)

The initial condition $m_0$ determines the shape of the growth curve at $t$=0:

* For $0<m_0<m_i$ (inflection point) curve starts as concave (curve 1, Figure 3);
* For $m_0 = m_i$ curve starts as linear (curve 2, Figure 3):
* For $m_i < m_0 < m_e$ curve starts as convex (curve 3, Figure 3).

Let us turn our attention now to cell growth by assuming that its global bio-synthesis can be described by a multitude of well synchronized processes described by equation (8), so that the cell mass (at least its bio-synthesizing component) will also be represented by Equation (8). Further, let us assume that the cell commitment to division is governed by a (still hypothetical, with some candidates being currently investigated) protein $U$. Protein $U$ is synthesized on a membrane of the size proportional to the total cell mass. Protein $U$ undergoes rapid turnover. It binds to a nucleus-localized receptor. Once the nucleus receptor is saturated, excess $U$ accumulates outside the nucleus and triggers the cell for the next cycle (crossing over the restriction point into S-phase).

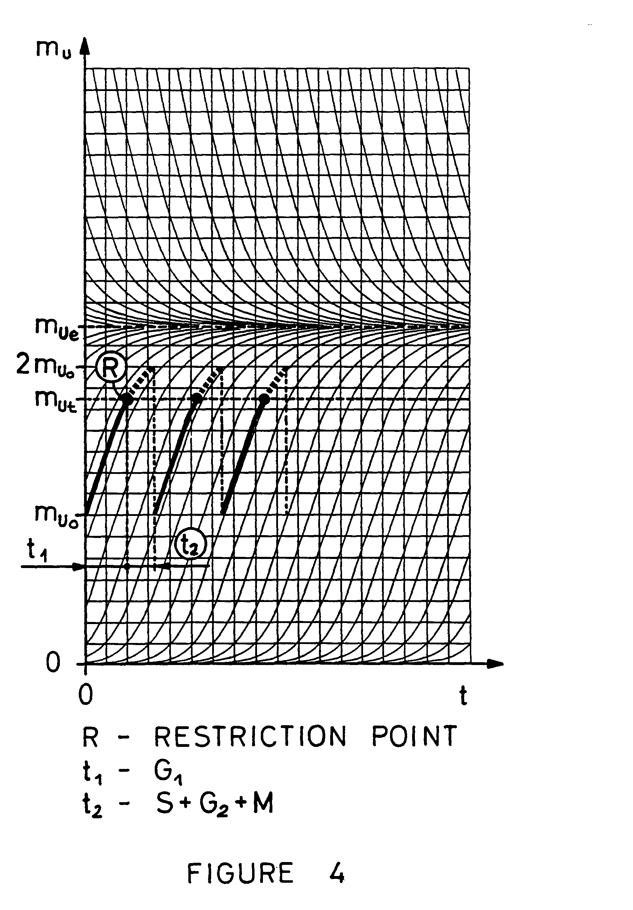

With above assumptions we can state that the quantity $m_U$ of $U$ present in the cell will be determined by equation (8) (assumption of rapid turnover leads to quantity of $U$ being simply proportional to the size of the synthesizing region). Commitment to division is then described by the condition that $m_U$ exceeds a threshold level $m_{Ut}$ Figure 4. The crossing would correspond to passing the restriction point R, i.e. entry into S-phase. The growth function past the point **R** might be different - on Figure 4, and all those that follow, it is shown as a simple extension (dashed line).

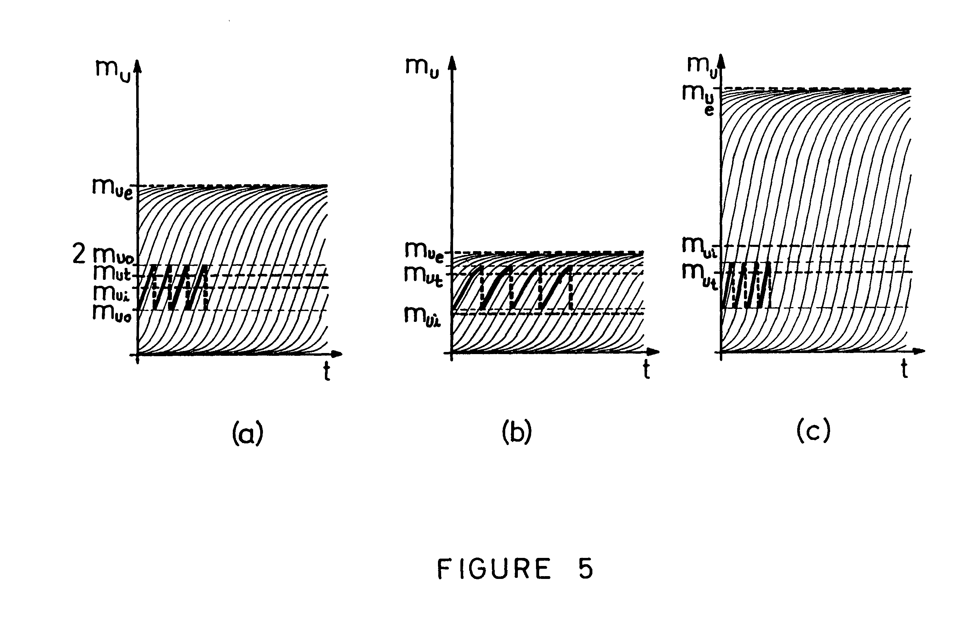

The shape of the growth curve will depend on the relation of $m_{Ue}$ to $m_{Ut}$. Figures 5a to 5c show three possibilities:

(i) If the cell cycle portion of the growth curve is centered on the inflection point $m_{Ui}$, i.e. $m_{Ui} = (m_{U0} + 2m_{U0})/2$, or $m_{U0} = 2/3m_{Ui}$, growth will be linear for all practical considerations, Figure 5a. The best-fit line through that section of the curve gives coefficient of determination of $R^2 = 0.99993$ ! That explains why the measurements of amino acid incorporation, done with full growth support, show constant rate throughout the cycle (and suggests that under those conditions the cycle is nearly centered on the inflection point).

(ii) Figure 5b shows a convex shape of the cell cycle portion of the growth curve with $m_{U0} > m_{Ui}$. The convexity is more pronounced as $m_{Ue}$ approaches $m_{Ut}$ (a slowly expanding culture).

(iii) Figure 5c shows a cell cycle on the concave part of the growth curve with $2m_{U0} < m_{Ui}$. This requires a high value of $m_{Ue}$ with respect to $m_{Ut}$. The limit on $m_{Ue}$ may make it difficult to establish this condition.

An interesting situation develops if $m_0 > m_e$. The cell should shrink to $m_e$. Now, it is reasonable to expect that for the vital functions the cell needs a minimum amount of mass (protein) - should the $m_e$ be pulled bellow this minimum, the cell will die on its way to $m_e$.

**Controllability**

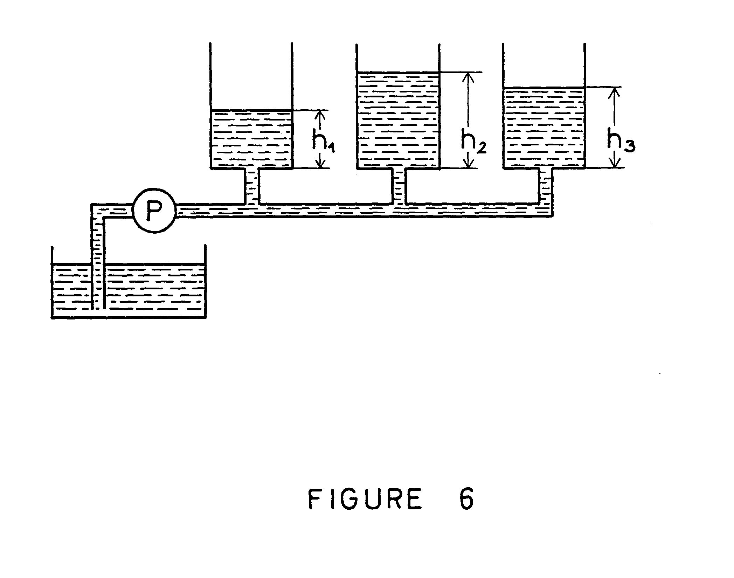

In this section a brief introduction to the concept of controllability is given by way of an illustration. Figure 6 shows a simple linear dynamic system comprising three fluid tanks connected by piping and a single pump to a larger fluid reservoir. Each of the tanks as seen from the pump has a time constant which is the product of the resistance to flow and the capacitance of the tank. If the three time constants are different one can show that the system is point-wise controllable. That means that it can be transferred from any initial state, defined in this case by the three levels of fluid in the tanks, to any final (desired) state in a finite time by some control action, in this case by the work of that single pump. In fact, any number of tanks connected to the pump in the way shown, provided the time constants are all different, is also controllable. Formally, this can be checked by a controllability criterion :

A linear system

$$\frac{d}{dt}(\underline{x}) = \underline{A}\ \underline{x} + \underline{B}\ \underline{u}$$

$$\underline{y} = \underline{C}\ \underline{x}$$

where $\underline{x}$ is the state vector of length **n**, $\underline{u}$ is the control vector of length **r,** and $\underline{y}$ is the output vector of length **m,** of the system defined by the matrices $\underline{A}$, $\underline{B}$ and $\underline{C}$ , is controllable if and only if the **n** x **nr** composite matrix [$\underline{B}$, $\underline{A}\ \underline{B}$, $\underline{A}^2\underline{B}$, ... ,$\underline{A}^{n-1}\underline{B}$] is of rank **n**.

Any number of tanks that would have the same time constant would behave in the same way, as the pump is used to pump the fluid in and out of the reservoir - they would not be independently controllable. For non-liner systems criteria of controllability are more complicated and will not be shown here, in spite of the fact that cells as modelled above do show strong non-linearity in the dynamics of growth. The example with tanks presented here is believed to serve as an important analogue to the problem at hand. It is rather counterintuitive to accept the possibility of controlling those tanks with a single pump. We shall make good use of the fluid tanks example in contemplating strategies for **selective** tumor depletion.

**Cell as a capacitance**

In the growth model of the cell we have seen that the equilibrium size of the cell is a function of the precursor concentration:

$$m_{e2} = [\frac{1}{\rho}(\frac{c}{\gamma} - \frac{1}{\alpha})]^{2/3};$$

the higher the concentration **c**, the higher the equilibrium size $m_{e\,2}$. Influence of the other constants is easy to appreciate as well - the equilibrium size (ultimately the amount of the trigger protein **U**) is increased by: (i) higher synthesis constant $\alpha$; (ii) lower turnover constant $\gamma$; and (iii) lower transport resistances $\rho$. There are good indications that the process of transformation leads to some or all of the contributing factors to push the $m_{e\,2}$ higher. The enormous complexity of the bio-synthesis process, whereby tens of thousands of substances are produced in a fully coordinated fashion, should not scare one away from taking a global view in modelling the cell - equation (8) does very well in showing the basic features of total cell mass dynamics. Consider a non-dividing cell in equilibrium, whereby the amount of products it is able to synthesize under the conditions given is just enough to compensate for the breakdown of those products. If the concentration of the precursor, and we shall consider only one - the critical, or limiting one of all those going into the process of synthesis, is changed to a lower value $c_{new}$, the cell will reduce its mass to the new value determined by $m_{e2}(c_{new})$. In doing so it will behave very much as a fluid tank changing its level in response to a change in pressure in the supply line. Nonlinearity of the response, by which the cell response differs from that of a simple tank, will depend on the step size. If the concentration of the critical precursor is now increased, the cell will respond by a mass increase. Most of the mass is protein and the building blocks are 20 amino acids (**lysine, arginine** and **histidine** with basic side chains; **aspartic** and **glutamic acid** with acidic side chains; **asparagine, glutamine, serine, threonine** and **tyrosine** with uncharged polar side chains; **glycine, alanine, valine, leucine, isoleucine, proline, phenylalanine, methionine, tryptophan** and **cysteine** with nonpolar side chains). Ten of those (arginine, threonine, **methionine, lysine, valine, leucine, isoleucine, histidine, phenylalanine** and **tryptophan**) are essential for vertebrates, i.e. they cannot be synthesized from any other substances and thus must be taken through diet (arginine can be synthesized, but apparently not in sufficient amounts). While any of the amino acids could be selected as a control parameter for the cell growth - lack of any single one totally inhibits protein synthesis - taking a nonessential one may require stronger control to fight cells' ability to compensate by increased synthesis. This strongly suggests controlling the concentration of one of the essential amino acids. Amino acids are free to cross the cell membrane and the internal pool, with some exceptions, is most of the time near equilibrium with the extracellular fluid, which in turn is near equilibrium with the blood levels. Thus controlling the level of at least one, and preferably of only one, essential amino acid in the blood circulation should give an effective control input to the cell protein synthesis process.

**Strategy**

Having presented a very simple controllable system which should serve only as a remote analogy to the problem at hand, we turn our attention to planing a strategy of attack on tumor cells. The goal is clear: to control the conditions of systemic nutrient availability during a finite time in such a way as to selectively deplete the pool of tumor cells of essential products. The control problem is complicated by the fact that cell populations undergoing division are not synchronized-that is true for both the normal cells (bone marrow, intestines) and the tumor cells. If one were to use standard approach to control, the initial conditions should be known. The control variables are the masses of normal and cancer cells in a body. The control input is the concentration of an essential nutrient, an essential amino acid. The system is moved from an initial state to the desired final state by some control input, which in this case is the varying concentration of a selected essential amino acid over a finite time. Based upon the initial conditions and the desired final state, the optimal input function can be calculated. Bringing all the cells to synchronism (into the rest phase, for example) will define, or set, the initial conditions. So the first goal is to synchronize - a long sought condition that should be of great benefit to conventional treatments (chemotherapy, radiation therapy) as well. According to the model presented above the cells which are dividing have the "equilibrium" (not in the true sense since they are prevented from reaching it by the constant cycling) value $m_{Ue}$ of the trigger protein $U$ higher than the threshold level $m_{Ut}$. Since $m_{Ue}$ is determined by the equilibrium size $m_e$ of the cell, which in turn is determined by the concentration of the (critical) precursor $c$, we can use the concentration of the precursor $c$ to stop cell division. The most plausible explanation for the transformation is that, under the same conditions, including the precursor concentration $c$,

$$m_{Ue} / \text{tumor} > m_{Ue} / \text{normal}.$$

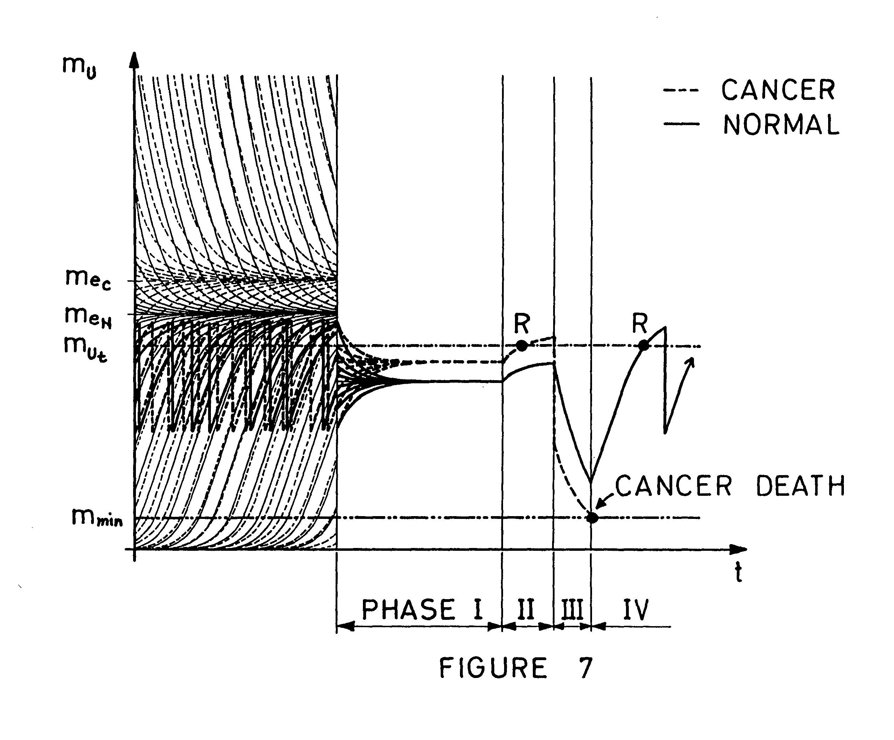

So if $c$ is lowered sufficiently for tumor cells to stop dividing, the normal ones will stop as well. Thus, in the first phase of the treatment, as shown on Figure 7, $c$ is to be decreased until all cells enter the rest phase, or $G_0$. Once this is done we can initiate further control with the known initial conditions. Preferably, the task of killing the tumor will be completed by control of the concentration of the chosen amino acid alone, but one could take an alternative approach and use this first step as synchronization only, followed by a conventional treatment such as chemotherapy. In the next, and most critically precise step, the concentration is increased to the level at which the tumor cells will cycle, but the normal ones will not (this is the condition which would be most suitable for conventional treatments - the normal cells are protected in $G_0$ and the tumor cells could be attacked as they cycle through mitosis). The task of controlling the system is now going to be much simpler. Tumor cells have been pushed over the restriction point and must complete the next division or otherwise die. So after a time of holding the concentration at this critical level, in the third phase it is decreased to the lowest possible level (caring to avoid systemic collapse due to lack of proteins). All of the cells are now loosing their mass, but the normal ones started much bigger - from the $G_0$ phase of the cycle - while the tumor ones have just completed a division ($M$ phase) and have a mass of perhaps 2/3 of the normal ones! Controllability of the system has been used to a great advantage - beyond what could be done with a linear system. After several hours tumor cells will run out of the minimum amount of protein needed, and die, while the normal cells are still safely above that limit. At the end, in the fourth phase, the concentration of the controlled amino acid is brought back to normal.

In all of above we have considered only the intrinsic parameters of cells (i.e. $\alpha$, $\gamma$, $\rho$ ), ignoring the external, additional influences past that of $c$. For example, it is clear that an external resistance on transport will influence the dynamics, by effectively increasing internal transport loses $\rho$. However, ignoring the external resistances can be justified in view of the micro-organization of tumors. They grow in globular structures with blood vessels attracted towards the globules and supplying the outer layers of dividing cells. Below the outer layer, or two, tumor cells are in the rest phase. The core, if big enough, is necrotic. Thus, targeting only the outermost layer adjacent to blood supply should suffice - any cells sandwiched between necrotic layers will die as well.

### Implementation

In this section a detailed description is given of the preferred implementation schemes of the strategy presented above. Two basic approaches to the treatment are possible: (1) to exert control over the concentration of the selected amino acid in an extracorporeal circulation apparatus resembling that used for dialysis; (2) to exert the control intracorporally, by decomposing or deactivating (masking) the selected amino acid by chemical means. Increasing the concentration in both cases is done by injecting the required amount of the amino acid. The first method appears more elegant, safer and probably allows for tighter control. The second one is more convenient to implement, but may cause stronger side effects.

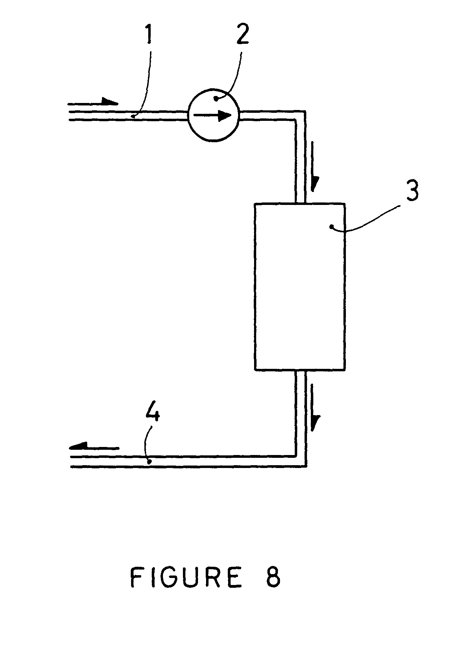

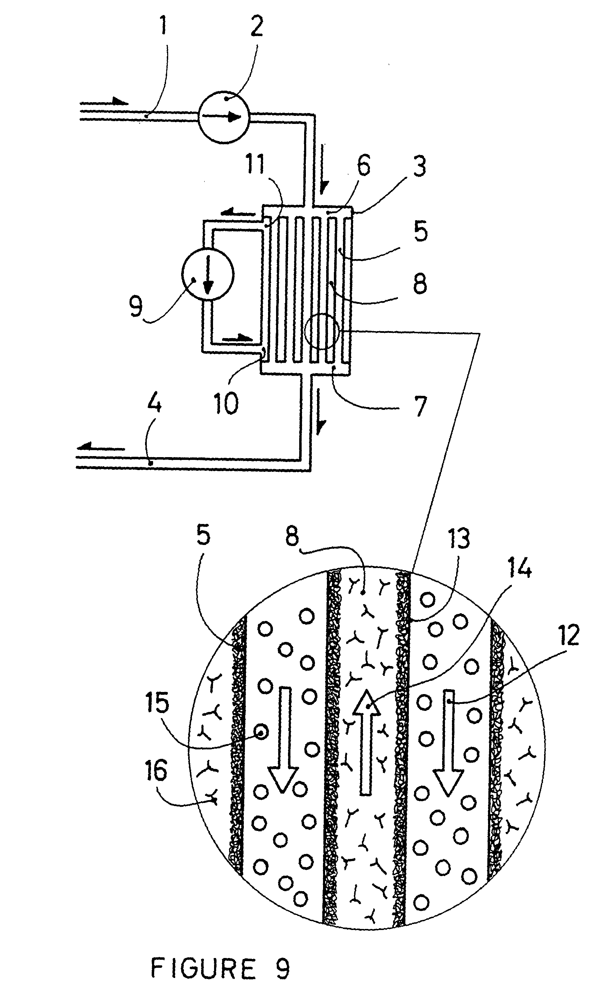

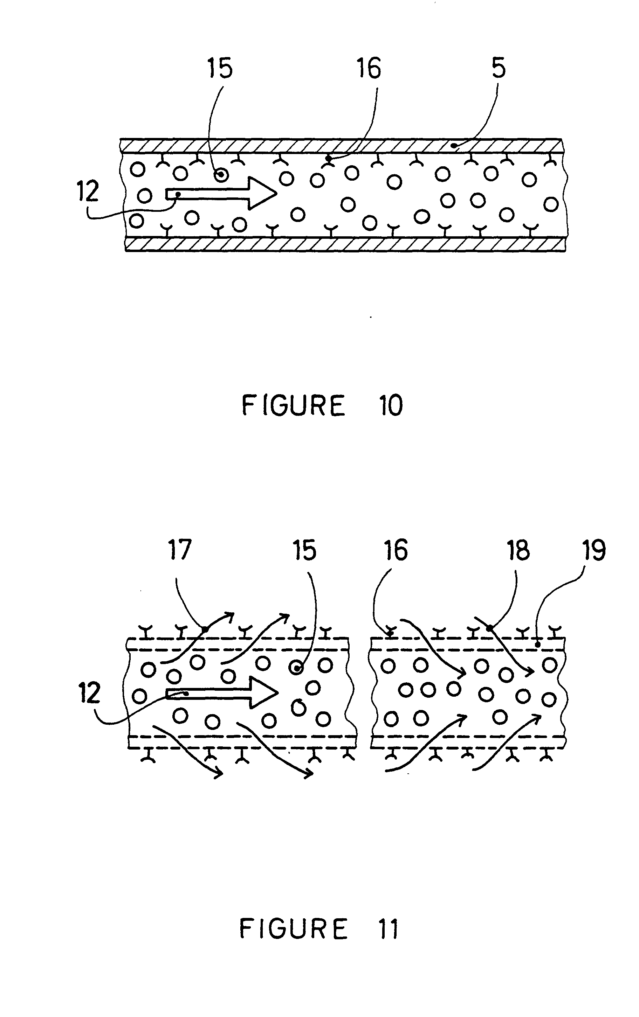

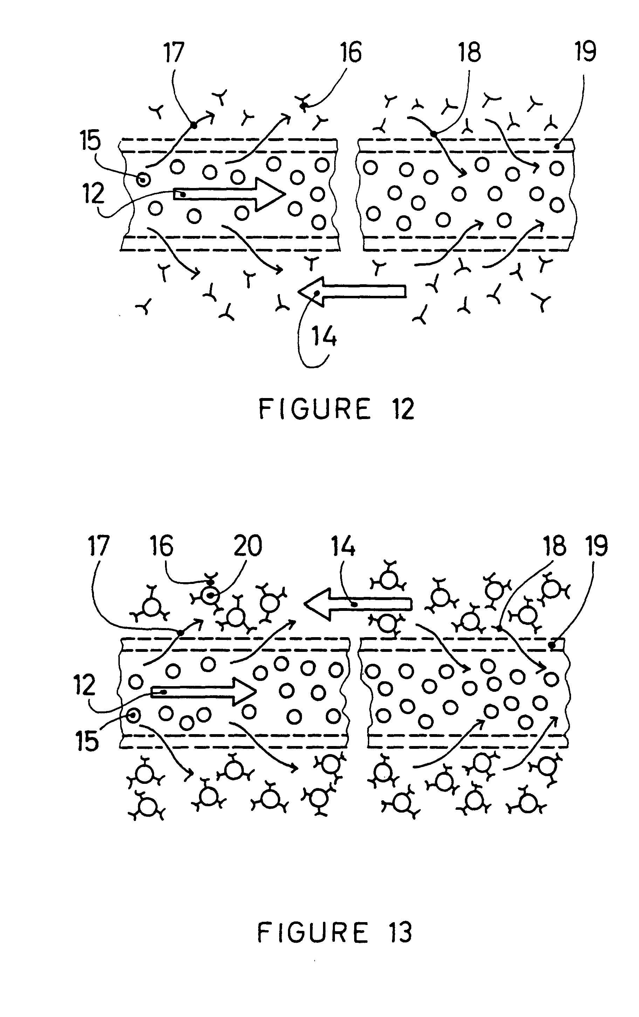

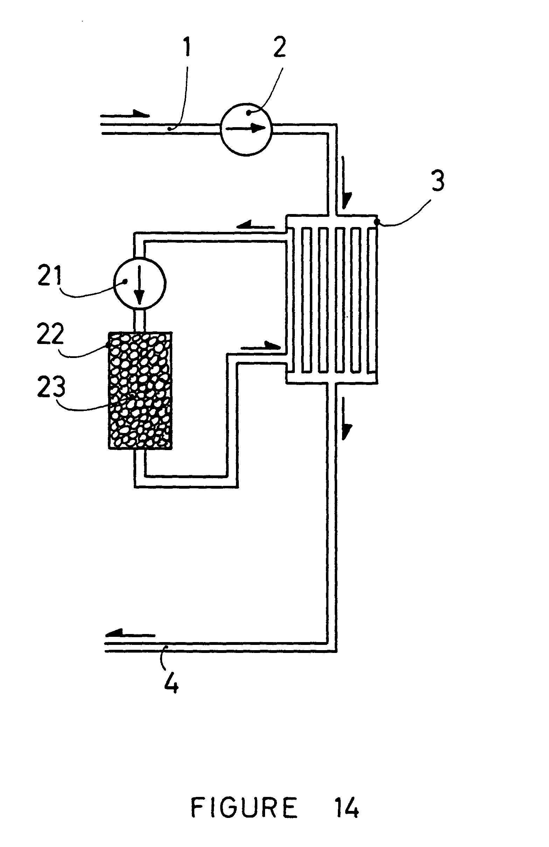

Extracorporeal control approach requires connections to an artery (femoral for example) and a vein and an interposed filter to remove the selected amino acid. Figure 8 shows an apparatus of one embodiment of the present invention in a simple configuration whereby the blood is taken out of arterial circulation through a tube 1, pumped by the pump 2 through a filter 3 and back into venous circulation through a tube 4. Figure 9 shows another embodiment of the present invention depicting an arrangement of apparatus components in more detail. The basic configuration remains the same for both adsorptive and decomposing approach to control. The filter 3 is a hollow fiber type with the blood moving through a set of tubes 5 connected in parallel at the inlet 6 and the outlet 7 of the filter. The extrafibrilar space between the hollow fibers (tubes) is denoted by 8 and is incorporated into another closed loop circulation driven by the pump 9. The flow through the extrafibrilar space 8 is directed in the opposite direction and the pressure drops are adjusted so that the pressure in the blood stream at the inlet 6 is higher than the pressure at the outlet 11 of the medium flowing through the space 8, which we shall refer to as external flow, while the pressure at the inlet 10 of the external flow is higher than the pressure at the outlet 7 of the blood flow. A detail of the hollow fiber 5 shows the blood flow 12 inside the fiber. Blood cells are denoted by 15. The wall 13 of the fiber is a molecular sieve allowing passage of only low molecular substances and water (with the cut-off at a few thousand Daltons, preferably 10 to 30 thousand). The external flow 14 through the space 8 is directed in the opposite direction. Figures 10 through 13 show different possibilities of removing the selected amino acid. All of these apply to both adsorption and decomposition, but one or another may be more suitable to the approach chosen. In Figure 10 an enzyme 16 is immobilized on the inner wall of the tubing 5. The blood flow 12 (blood cells are shown by 15) moves along the walls and the selected amino acid is either adsorbed or decomposed by the action of the enzyme 16. This is the least efficient method proposed - the surface available for enzyme immobilization is very limited and the fluid is stationary along the walls, bringing the substances in contact mostly by diffusion. Additionally, blood is in direct contact with the enzyme which if released would enter the flow, but also be exposed to unwanted interactions with high-molecular components of the blood plasma, perhaps even with the blood cells. Since the walls are impermeable, the external circulation (driven by pump 9) is not needed. Figure 11 shows an improvement with the fiber walls 19 allowing passage of water and small solutes. Due to the pressure gradients set up as described above, the fluid flows out from the tubes at the inlet of blood stream as shown by the arrows 17 and returns at the outlet as shown by the arrows 18. The active enzyme 16 is immobilized on the outer wall of the tube 19. A known hollow fiber technology exists whereby the outside of the fiber is made porous. Such tubing would offer a bigger surface to be covered by the active enzyme. Figure 12 shows an arrangement where the external flow 14 carries the enzyme 16 in solution. This increases the chances of selected amino acid/enzyme interaction. As before, the cross flow is out of the blood stream at the inlet, arrow 17, and back into the blood stream, arrow 18, at the outlet of the filter. Figure 13 shows a modification of the arrangement shown on Figure 12 inasmuch the enzyme 16 is immobilized on cross-linked gel beads 20 being pumped in the external circulation 14. Handling of such a system at times of enzyme saturation (if adsorption is used) is superior to the free solution system of Figure 12. Figure 14 shows a modification, where the external flow is now pumped by a pump 21 through a gel-bed type filter 22 with the gel-bound enzyme 23. The external flow again contains only the low-molecular components of blood plasma filtered in the first stage filter 3. The pump 21 can compensate for the high pressure drop in the filter 22 tightly packed with the gel beads. Pressures at the inlet and outlet of the external flow can be additionally controlled to keep the conditions of the cross flow in the hollow fiber filter 3 as explained on figure 9. Tightly packed gel filter 22 will yield higher amino acid removal efficiency than dispersed gel beads flowing in the external circulation of figure 9.

The higher the single filter pass removal ratio, the higher the speed of control of the selected amino acid concentration on the whole body level. An estimate of the time constant for control can be made as the ratio of the "holdup" (total amount of amino acids in the body) to "throughput" (amino acid mass flow through the extracorporeal filter). If the efficiency of the filter was 100%, i.e. assuming total removal in a single filter pass, time constant with the femoral artery tap would be on the order of two to four hours - sufficiently fast for the control requirements against cell cycle duration of about 24 hours.

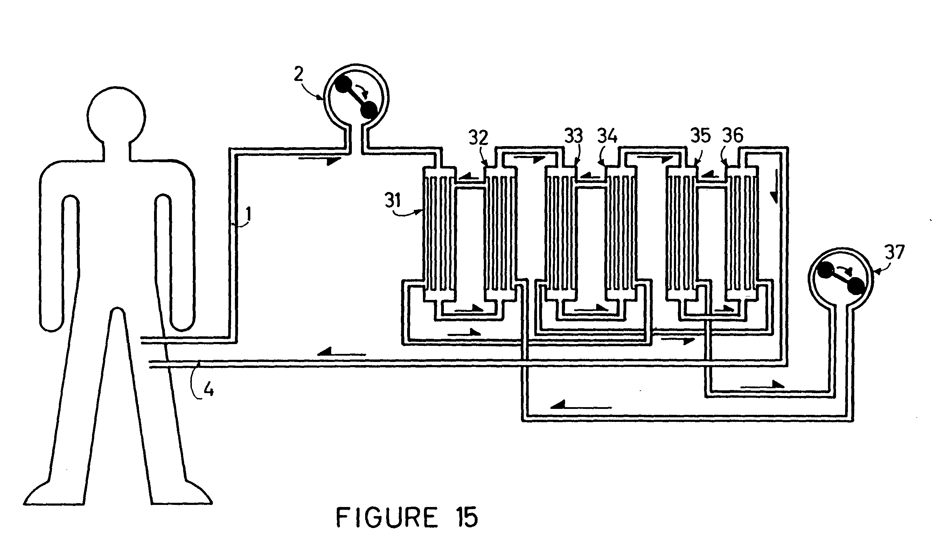

Due to increase in viscosity of the blood by removal of the plasma fluid, the efficiency of the filter arrangements depicted above is limited to about 10% in a single stage. Multiple stages can be utilized as depicted on Figure 15. In this arrangement low molecular components of blood plasma are pushed out from the blood in the first filter 31, reacted with the enzyme(s), and returned to the blood stream in the second filter 32. This is repeated in the third and fourth filters, 33 and 34, respectively, and then again in the fifth and sixth filters, 35 and 36. Enzyme(s) are pumped by the pump 37 in parallel with the blood in order to maintain approximately constant pressure difference along the filters. This leads to better utilization of the filter. With three stages of two filters each, the total efficiency of amino acid removal is about 50%, increasing the time constant to four to eight hours.

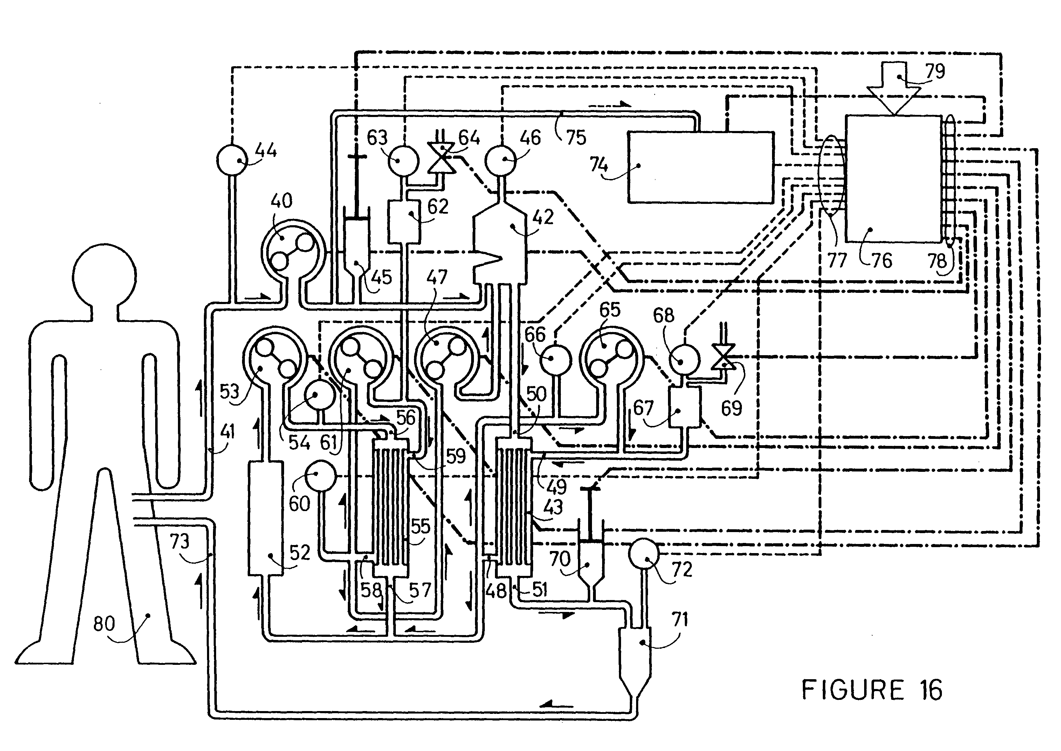

Figure 16 shows another configuration of filters, whereby the efficiency of removal is greatly enhanced by dilution, or "washing", of the blood prior to filtration. A peristaltic blood pump 40 moves the blood from the arterial blood line 41, through an expansion chamber 42, into the first blood filter 43, which is preferably of a fiber-type.

Arterial line pressure is measured by the gauge 44. Heparin, which may be used for anticoagulation, is injected into blood stream by an injection pump 45. Filter inlet pressure is measured in the expansion chamber by the gauge 46. Another pump 47 pumps dilution fluid into the expansion chamber 42 where it is mixed with the blood prior to entry into the filter 43 through the inlet port 50. All of the flow added to the blood by the pump 47 is removed by filtration in the filter 43. Filtrate is removed via the jacket port 48, and partially recirculated by the pump 65. Inlet pressure to the pump 65 is measured by the gauge 66. Pressure in the expansion chamber 67 is measured by the gauge 68 and controlled by the valve 69 connected to a pressure source. In this way the pressure drop from the inlet jacket port 49 to the outlet jacket port 48 can be controlled, as well as the pressure at the port 49. This allows for matching the pressure profile outside the fibers along the filter length to the pressure profile inside the fibers, and thus the most efficient utilization of the filter area. The filtrate, i.e. the dilution fluid, is moved from the jacket port 48 to a reactor chamber 52. On the way to the rector, this filtrate is mixed with the enzyme-carrying fluid emanating from the outlet 57 of the second filter 55. Enzyme-carrying fluid is pumped from the reactor 52 by the pump 53 back into the filter 55 via the inlet port 56. Output pressure of the pump 53 is monitored by the gauge 54. Enzyme(s) action is exerted on the amino acid(s) filtered from the blood in the filter 43 during the time the mixture is held in the reactor 52. In the filter 55 enzyme(s) are confined to the inside of filter fibers while the fluid carrying the reaction products is filtered out and removed via the jacket port 58. From here it is pumped back into blood stream by the pump 47. Inlet pressure for the pump 47 as well as for the pump 61 is measured by the gauge 60. The purpose of the pump 61 for the filter 55 is the same as that of the pump 65 for the filter 43 -- to control the profile of pressure drop within the filter jacket. Output pressure of the pump 61 is monitored and controlled by the gauge 63 and the valve 64 attached to the lines via the expansion chamber 62. The blood flow rate at the outlet 51 of the filter 43 is the same as that through the blood pump 40, i.e. there is no change of blood volume in the machine. The concentration of the amino acid being controlled at the blood filter outlet 51 depends on the ratio of dilution fluid being added to the blood prior to filtration. For a ratio of the blood flow to the dilution fluid flow of 1:2, concentration at the outlet is about one third of that at the inlet. If this is not sufficient, one can either increase this ratio, or send the blood through another, identical filtration stage (less the blood pump). In two filtration stages, approximately 90% removal of the amino acid is possible by this arrangement. This is clearly a superior embodiment to the one depicted on Figure 15, whereby the blood was passing through six filters for only a 50% removal. The additive effects of diffusion across the filter may increase efficiencies of both configurations. Some enzyme(s) will leak across the fiber walls of filter 55 and into the blood stream, but this is controllable by filter selection -- in general 30 kDaltons cut-off is sufficient to retain most enzymes considered. After exiting from the filter 43 blood stream may be injected by an amino acid by the injection pump 70. Prior to return via venous line 73, blood passes through the bubble catcher 71. The pressure in the return line is monitored by the gauge 72.

All of the pressure gauges are monitored by the computer/controller 76. It also receives data from the amino analyzer 74, which on request by the computer/controller takes a blood sample via blood line 75. All computer/controller input data lines 77 are marked by dashed lines. All outputs 78 from the computer/controller are marked by dash-period-lines. They are setting control parameters to all pumps as requested by the feedback regulation of the amino acid concentration at the inlet to the apparatus (which is equal to systemic levels) in order to follow a pre-programmed sequence 79 supplied to the computer/controller 76. The patient 80 may be continuously connected to the apparatus for the best possible control, but the same apparatus may be used to process the blood in small batches. Single-needle hemodialysis is an example of such small batch processing.

Pumps and filters must be designed to minimize damage of the blood cells. The technology used for dialysis and blood oxygenating machines can be readily applied. The only specific problem in this case is the enzymatic system to remove the selected amino acid.

Amino acids which are not utilized for synthesis of proteins and other nitrogen-containing compounds enter various metabolic pathways whereby they get converted into mostly energy supplying substances. The liver and muscles, and to a lesser extent brain and kidneys are the main sites of amino acid degradation. While it might be possible to use plant, or microbial degradation enzymes, it appears that the best, and safest approach is to chose a pathway occurring naturally in mammals. In selecting one of a great number of possibilities consideration should be given to several issues: selectivity of the enzymatic action; possible toxicity of the byproducts; availability, purity and the cost of the enzyme; cofactors; energy requirements, etc. The following list is by no means comprehensive - it simply lists some possibilities considered acceptable in view of the criteria above. If **threonine** is selected for control, ***threonine aldolase*** can be used to degrade threonine into glycine and acetaldehyde. For **phenylalanine** the enzyme ***phenylalanine hydroxylase*** can be used to convert it into **tyrosine -** cofactor is **DL-6-methyl-5,6,7,8-tetra-hydropterine. Arginine** can be catabolized to **urea** and **ornithine** by ***arginase.* Histidine** can be converted to **urocanic acid** by ***histidine ammonia-lyase.* Tryptophan** is degraded by ***tryptophan 2,3-dioxygenase*** into **N-formylkynurenine.**

In the natural process of protein assembly amino acids are first attached to a specific tRNA molecule. The process of attachment is highly specific, is well understood and suggests itself as a model for the adsorption filter design. Several possibilities will be described and are to be used with the filter arrangements shown on figures 9 and 11 to 14. Any one of the amino acids selected can be removed from the blood in this manner.

An amino acid attachment to its corresponding tRNA proceeds in two steps, both catalyzed by the same enzyme ***aminoacyl-tRNA synthetase*** and powered by **ATP** (adenosine triphosphate). In the first step the amino acid is activated by formation of an ***aminoacyl-adenylate*** (also called ***aminoacyl-AMP;*** AMP for adenosine monophosphate) from an amino acid and ATP. In the absence of the corresponding tRNA, the aminoacyl-AMP intermediate is a stable molecule and does not dissociate from the synthetase. This points to the first possibility for the removal of the selected amino acid - use of the corresponding aminoacyl-tRNA synthetase and ATP in the external circulation of the filter according to figures 9, 11 to 13, or the external flow in the filter 22 of figure 14. In the external flow of figure 9, the enzyme may be used free in solution (molecular weight of these synthetases is on the order of 100 to 200 kDaltons and can be easily kept isolated from the blood flow behind the molecular sieve of the hollow fiber filter) or bound to a crosslinked gel. Immobilizing on the crosslinked agarose gel can be done for example with a commonly used ligand such as **CIBACRON BLUE F3GA.** ATP is added to the external circulation as needed.

In the second step the aminoacyl group of the aminoacyl-AMP is transferred to a tRNA to form *aminoacyl-tRNA* which is an active intermediate in the protein synthesis. This is a further possibility of removal of the selected amino acid from the blood stream. If the corresponding tRNA is supplied the enzyme aminoacyl-tRNA synthetase can be used only to couple the amino acid to tRNA instead of binding it itself. Relevant filter configurations are shown on figures 9 and 11 to 14. Both the selected aminoacyl-tRNA synthetase and the corresponding tRNA can be supplied to the external circulation in solution. Alternatively, the enzyme can be gel-immobilized and tRNA supplied in solution, or tRNA can be gel-immobilized and the enzyme supplied in solution. All of the intermediates and the products are large enough to remain isolated from the blood flow behind the molecular sieve.

Searches of scientific and patent literature have revealed several references containing relevant information. No prior attempt has been made to devise a dynamic regimen of treatment aimed at selective depletion of protein in cancer cells, or the apparatus to carry out such a program. Abstracts of the papers included hereby are provided as an evidence of positive effects that restriction on amino acid supply can have on tumor growth. Most relevant from the patent literature is the U. S. Patent No. 4,955,857 by Shettigar. It describes a blood filter loaded by selected degradative amino acids enzymes. The aim of that invention was to minimize side effects of intravenous enzymes' injections used for tumor treatment, particularly for certain leukemia types.

AN 92082371.

AU Yeatman-T-J, Risley-G-L, Brunson-M-E.

IN Department of Surgery, University of Texas, MD Anderson Cancer Center, Houston 77036.

TI Depletion of dietary arginine inhibits growth of metastatic tumor.

SO Arch-Surg 1991 Nov, VOL: 126 (11), P: 1376-81; discussion 1381-2, ISSN: 0004-0010.

AB The effects of dietary arginine on the growth of a murine colon tumor metastatic to the liver were examined in a model of advanced neoplastic disease. Tumor growth was influenced by arginine both in vivo and in vitro. An arginine-supplemented diet stimulated tumor growth by 55% compared with controls. Conversely, an argininedepleted diet inhibited tumor growth by 78% compared with controls. In vitro culture of both murine and human colon tumor cells confirmed that arginine was necessary for cell growth. Flow-cytometric analysis using propidium iodide and bromodeoxyuridine suggested that colon tumor cells cultured without arginine enter a quiescent S phase and depend on arginine for further growth and cell cycle progression. The potential roles for selective dietary arginine modulation in patients with cancer with advanced disease are discussed. Author.

AN 91331208.

AU Taylor-M-W, Feng-G-S.

IN Department of Biology, Indiana University, Bloomington 47405.

TI Relationship between interferon-gamma, indoleamine 2,3-dioxygenase, and tryptophan catabolism (see comments).

SO FASEB-J 1991 Aug, VOL: 5 (11), P: 2516-22, ISSN: 0892-6638 60 Refs. CM Comment in: FASEB-J 1991 Nov; 5(14):3003-4.

AB Interferons have been shown to be potential anti-cancer agents and to inhibit tumor cell growth in culture. The in vivo mechanism of the anti-proliferative effect may be direct or indirect through the immune system; however, in vitro a primary mechanism of cytotoxicity is through the depletion of tryptophan. In particular, interferongamma (IFN-gamma) induces an enzyme of tryptophan catabolism, indoleamine 2,3-dioxygenase (IDO), which is responsible for conversion of tryptophan and other indole derivatives to kynurenine. The inhibitory effect of interferon on many intracellular parasites such as Toxoplasma gondii and Chlamydia trachomatis is by the same mechanism. Elevated kynurenine levels have been found in humans in a number of diseases and after interferon treatment, and the enzyme is induced in rodents after administration of interferon inducers, or influenza virus. IDO induction also occurs in vivo during rejection of allogeneic tumors, indicating a possible role for this enzyme in the tumor rejection process. The gene for IDO has been cloned and shown to be differentially regulated by IFN-alpha and IFN-gamma. IDO induction has been correlated with induction of GTP-cyclohydrolase, the key enzyme in pteridine biosynthesis. A direct role for IDO in pteridine synthesis has not been shown, and this parallel induction may reflect coordinate regulation of genes induced by IFN-gamma. A possible role for IDO in O2-radical scavenging and in inflammation is discussed. Author.

AN 92125173.

AU Brown-R-R, Ozaki-Y, Datta-S-P, Borden-E-C, Sondel-P-M, Malone-D-G. IN Department of Human Oncology, University of Wisconsin Medical School, Madison 53792.

TI Implications of interferon-induced tryptophan catabolism in cancer, auto-immune diseases and AIDS.

SO Adv-Exp-Med-Biol 1991, VOL: 294, P: 425-35, ISSN: 0065-2598 77 Refs.

AB Tryptophan (Trp) is an indispensable amino acid required for biosynthesis of proteins, serotonin and niacin. Indoleamine 2,3-dioxygenase (IDO) is induced by infections, viruses, lipopolysaccharides, or interferons (IFNs) and this results in significant catabolism of Trp along the kynurenine (Kyn) pathway. Intracellular growth of Toxoplasma gondii and Chlamydia psittaci in human fibroblasts in vitro is inhibited by IFN-gamma and this inhibition is negated by extra Trp in the medium. Similarly, growth of a number of human cell lines in vitro is inhibited by IFN-gamma and addition of extra Trp restores growth. Thus, in some in vitro systems, antiproliferative effects of IFN-gamma are mediated by induced depletion of Trp. We find that cancer patients given Type I or Type II IFNs can induce IDO which results in decreased serum Trp levels (20-50% of pretreatment) and increased urinary metabolites of the Kyn pathway (5 to 500 fold of pretreatment). We speculate that in vivo antineoplastic effects of IFNs and clinical side effects are mediated, at least in part, by a general or localized depletion of Trp. In view of reported increases of IFNs in autoimmune diseases and our earlier findings of elevated urinary Trp metabolites in autoimmune diseases, it seems likely that systemic or local depletion of Trp occurs in autoimmune diseases and may relate to degeneration, wasting and other symptoms in such diseases. We find high levels of IDO in cells isolated from synovia of arthritic joints. IFNs are also elevated in human immunodeficiency virus (HIV) patients and increasing IFN levels are associated with a worsening prognosis. We propose that IDO is induced chronically by HIV infection, is further increased by opportunistic infections, and that this chronic loss of Trp initiates mechanisms responsible for the cachexia, dementia, diarrhea and possibly immunosuppression of AIDS patients. In these symptoms, AIDS resembles classical pellagra due to dietary deficiency of Trp and niacin. In preliminary studies, others report low levels of Trp and serotonin, and elevated levels of Kyn and quinolinic acid in AIDS patients. The implications of these data in cancer, autoimmune diseases and AIDS are discussed. Author.

AN 93251363.

AU Ho-D-H, Covington-W-P, Wallerstein-R-O, Hester-J-P, Lin-J-R, Brown-N-S, Newman-R-A, Krakoff-l-H, Freireich-E-J.

IN Division of Medicine, University of Texas, M. D. Anderson Cancer Center, Houston 77030.

TI Depletion of patients' plasma tryptophan using tryptophan side-chain oxidase columns.

SO Cancer-Invest 1993, VOL: 11 (3), P: 252-7, ISSN: 0735-7907.

AB The use of the enzyme tryptophan side-chain oxidase, isolated from Pseudomonas XA, was explored in 3 patients with refractory acute lymphocytic leukemia. Patients were given either a low-tryptophan diet or tryptophan-free hyperalimentation, prior to and during therapy. Their plasma, separated by pheresis, was continuously passed through a tryptophan depletion column containing the immobilized tryptophan side-chain oxidase. Up to 4 plasma volumes were passed through the column daily, 5 days per week for 2-3 weeks, and plasma tryptophan levels, both free and total, were measured by high-performance liquid chromatography. Pre- and postcolumn plasma samples were collected throughout the pheresis procedure. All postcolumn plasma samples had unmeasurable tryptophan levels throughout the treatment period, whereas precolumn samples were always measurable. Generally, tryptophan levels of plasma isolated from peripheral blood decreased after therapy, but rebounded by the next day. The enzyme depletion column reduces circulating plasma tryptophan levels, and its use is well tolerated by patients. However, further development of this method will require study of the effects of diet and of the duration, interval, and frequency of use of this column on therapeutic efficacy. Problems include difficulties with extended diet compliance and apparently intensive mobilization of tryptophan from body stores, which may preclude the clinical application of this enzyme depletion column. Author.

## Claims

1. Blood treatment apparatus comprising means (2) for moving blood from the inlet port (1) to the outlet port (4) of said apparatus
and means (74) for measuring the concentration of an essential amino acid in said blood,
**characterized by**

   A) means (3) for reacting low molecular components of said blood with a chemical agent which reduces concentration of at least one of the essential amino acids arginine, threonine, methionine, lysine, valine, leucine, isoleucine, histidine, phenylalanine or tryptophan, in said blood,
   B) means (70) for increasing said concentration by addition of said at least one of said essential amino acids to said blood, and
   C) means (76) for executing a pre-programmed sequence of changes of said concentration.

2. Apparatus according to claim 1, whereby said means (2) for moving said blood comprise at least one extracorporeal blood pump, preferably of a peristaltic type, and suitable blood lines.

3. Apparatus according to claim 1 or 2, whereby said means for reacting low molecular components of said blood with said chemical agent comprise at least one molecular sieve filter which separates said chemical agent from said blood, whereby said low molecular components of said blood, including said amino acids, can move across said filter and react with said chemical agent.

4. Apparatus according to one of the claims 1 to 3, whereby said chemical agent is able to reduce said concentration by selective adsorption of at least one of said essential amino acids.

5. Apparatus according to one of the claims 1 to 3, whereby said chemical agent is an enzyme which is able to reduce said concentration by selective decomposition of said amino acids.

6. Apparatus according to one of the claims 1 to 5, whereby said means (70) for increasing said concentration by addition of said essential amino acids to said blood comprise an injection pump.

7. Apparatus according to one of the claims 1 to 6, whereby said means (74) for measuring said concentration of said essential amino acids in said blood comprise an amino acids analyzer.

8. Apparatus according to one of the claims 1 to 7, whereby said means (76) for executing a pre-programmed sequence of change of said concentration comprise at least one computer controlling and receiving data from said means for measuring said concentration of said essential amino acids; controlling said means for moving said blood through said means for reacting said low molecular components of said blood with said chemical agents; and controlling said means for increasing said concentration of said essential amino acids.

9. Apparatus according to one of the claims 1 to 8 whereby said pre-programmed sequence spans a finite time in the range of one to seven days.

10. Apparatus according to one of the claims 1 to 9 whereby said pre-programmed sequence comprises at least four stages, whereby said concentration at the first level during the time of the first stage causes all cells in the body to enter the rest phase, and said concentration at the second level during the time of the second stage allows tumor cells to re-enter cycle, but does not allow normal cells to re-enter cycle, and said concentration at the third level during the time of the third stage causes majority of cycling tumor cells to die, and said concentration at the fourth stage is the normal concentration allowing normal cells to resume cycling.

11. Apparatus according to one of the claims 1 to 10, whereby said means for reacting comprises an extracorporeal blood filter (3) and a tubing to connect said extracorporeal blood filter (3) and said extracorporeal blood pump (2) into a closed extracorporeal circulation between an artery and a vein, whereby said extracorporeal blood filter selectively eliminates said amino acid from the blood.

12. Apparatus according to claim 11, whereby said filter (3) selectively adsorbs said amino acid.

13. Apparatus according to claim 11 or 12, whereby said selective adsorption is achieved by aminoacyl-tRNA synthetase specific to said amino acid and is powered by ATP.

14. Apparatus according to claim 13, whereby said aminoacyl-tRNA synthetase is bound to a gel.

15. Apparatus according to one of the claim 11 or 12, whereby, said selective adsorption is achieved by tRNA specific to said acid and is catalyzed by aminoacyl-tRNA synthetase specific to said amino acid and is powered by ATP.

16. Apparatus according to claim 15 whereby said tRNA is bound to a gel.

17. Apparatus according to claim 15 whereby said aminoacyl-tRNA synthetase is bound to a gel.

18. Apparatus according to claim 11, whereby said filter (3) selectively decomposes said essential amino acid.

19. Apparatus according to claim 18, whereby said decomposition is achieved by one of the natural amino acid degradative enzymes.

20. Apparatus according to claim 19, whereby said enzymes are selected from the following: threonine aldolase for threonine, phenylalanine hydroxylase for phenylalanine, arginase for arginine, histidine ammonia-lyase for histidine, tryptophan 2,3-dioxygenase for tryptophan.

21. Apparatus according to claim 19, whereby said enzyme is bound to a gel.

22. Apparatus according to claim 11, whereby said blood filter (3) for said selective elimination of said amino acid from said blood incorporates a secondary external circulation flow separated from the blood flow by a molecular sieve.

23. Apparatus according to claim 22, whereby said selective elimination of said amino acid is achieved by enzymatic means limited to said secondary circulation.

24. Apparatus according to one of the claims 11 to 23, further comprising:

(a) a first extracorporeal filter of hollow fiber type;

(b) an extracorporeal blood-plasma fraction pump;

(c) a second extracorporeal filter of gel-bed type; and

(d) a tubing to connect to components (a), (b), (c) and to said extracorporeal blood pump so that in use arterial blood is pumped by said extracorporeal blood pump through said hollow fibers of (a) and returned into venous blood flow, while the low-molecular fraction of blood plasma which filters through said hollow fibers is pumped by (b) through (c) forming a secondary closed external circulation between (c) and the extrafibrilar space of (a), whereby said first filter separates blood cells and high-molecular weight substances from said blood-plasma fraction containing amino acids, and said second extracorporeal filter, supplied by said blood-plasma fraction pump, selectively eliminates said essential amino acid from said blood-plasma fraction.

25. Apparatus according to claim 24, whereby said second filter of gel-bed type selectively adsorbs said amino acid.

26. Apparatus according to claim 25, whereby said selective adsorption is achieved by aminoacyl-tRNA synthetase specific to said amino acid and is powered by ATP.

27. Apparatus according to claim 25, whereby said selective adsorption is achieved by tRNA specific to said amino acid and is catalyzed by aminoacyl-tRNA synthetase specific to said amino acid and is powered by ATP, and where either said tRNA or said aminoacyl-tRNA synthetase specific to said amino acid is gel-bound.

28. Apparatus according to claim 24, whereby said second filter of gel-bed type selectively decomposes said essential amino acid.

29. Apparatus according to claim 28, whereby said decomposition is achieved by one of the natural amino acid degradative enzymes.

30. Apparatus according to claim 29, whereby said enzyme is selected from the following: threonine aldolase for threonine, phenylalanine hydroxylase for phenylalanine, arginase for arginine, histidine ammonia-lyase for histidine, tryptophan 2,3-dioxygenase for tryptophan.

31. Apparatus according to one of the claims 24 to 30, further comprising:

(a) a first filter filtrate pump;

(b) a second filter of hollow fiber type;

(c) a second filter filtrate pump;

(d) a reactor chamber; and

(e) fluid lines to connect components (a), (b), (c), (d) and said extracorporeal blood pump and said first filter of hollow fiber type so that in use arterial blood is pumped by said extracorporeal blood pump through said first filter of hollow fiber type and returned into venous blood flow, while said filtrate of said first filter of hollow fiber type carrying low-molecular fraction of blood plasma is pumped by (a) through (d) and into said hollow fibers of said second filter (b), whereby the concentrate of said second filter (b) is mixed with said first filter filtrate prior to entry into (d), whereby said second filter filtrate is pumped by (c) to mix with said arterial blood to entry onto said first filter of hollow fiber type, whereby said first filter separates blood cells and high-molecular weight substances from said blood-plasma fraction containing amino acids, and said second filter separates enzymes which selectively eliminate at least one of said amino acids from a blood diluting fluid which is circulated between said to filters.

32. Apparatus according to claim 31, whereby recirculating fluid flow through jackets of said filters is controlled by additional pumps connecting the inlet and outlet ports of said jackets.

33. Apparatus according to claims 31 or 32, whereby said filters comprise means for controlling the average pressure within said jackets of said filters.


**Revendications**

1. Appareil de traitement du sang, comprenant des moyens (2) pour déplacer le sang depuis l'origine d'entrée (1) jusqu'à l'orifice de sortie (4) dudit appareil,
et des moyens (74) pour mesurer la concentration d'un acide aminé essentiel dans ledit sang,
caractérisé par

A) des moyens (3) pour faire réagir des composants à faible poids moléculaire dudit sang avec un agent chimique qui réduit la concentration d'au moins l'un des acides aminés essentiels arginine, thréonine, méthionine, lysine, valine, leucine, isoleucine, histidine, phénylalanine ou tryptophane dans ledit sang,

B) un moyen (70) pour augmenter ladite concentration par addition dudit acide aminé essentiel, au moins un, audit sang, et

C) un moyen (76) pour exécuter une séquence préprogrammée de modification de ladite concentration.

2. Appareil selon la revendication 1, dans lequel lesdits moyens (2) pour déplacer ledit sang comprennent au moins une pompe sanguine extracorporelle, de préférence du type péristaltique, et des conduits sanguins appropriés.

3. Appareil selon la revendication 1 ou 2, dans lequel lesdits moyens pour faire réagir des composants à faible poids moléculaire dudit sang avec ledit agent chimique comprennent au moins un filtre à tamis moléculaire qui sépare ledit agent chimique dudit sang, grâce à quoi lesdits composants à bas poids moléculaire dudit sang, notamment lesdits acides aminés, peuvent traverser ledit filtre et réagir avec ledit agent chimique.

4. Appareil selon l'une des revendications 1 à 3, dans lequel ledit agent chimique est capable de réduire ladite concentration par adsorption sélective d'au moins l'un desdits acides aminés essentiels.

5. Appareil selon l'une des revendications 1 à 3, dans lequel ledit agent chimique est une enzyme qui est capable de réduire ladite concentration par des compositions sélectives desdits acides aminés.

6. Appareil selon l'une des revendications 1 à 5, dans lequel lesdits moyens (70) pour augmenter ladite concentration par addition desdits acides aminés essentiels dans ledit sang comprennent une pompe d'injection.

7. Appareil selon l'une des revendications 1 à 6, dans lequel lesdits moyens (74) pour mesurer ladite concentration desdits acides aminés essentiels dans ledit sang comprennent un analyseur d'acides aminés.

8. Appareil selon l'une des revendications 1 à 7, dans lequel lesdits moyens (76) pour exécuter une séquence préprogrammée de modification de ladite concentration comprennent au moins un ordinateur qui commande et reçoit des données desdits moyens de mesure de ladite concentration en lesdits acides aminés essentiels ; qui commande lesdits moyens pour déplacer ledit sang à travers lesdits moyens pour faire réagir lesdits composants à faible poids moléculaire dudit sang avec lesdits agents chimiques; et qui commande lesdits moyens d'augmentation de ladite concentration en lesdits acides aminés essentiels.

9. Appareil selon l'une des revendications 1 à 8, dans lequel ladite séquence préprogrammée s'étend sur une durée finie de l'ordre d'un à sept jours.

10. Appareil selon l'une des revendications 1 à 9, dans lequel ladite séquence préprogrammée comprend au moins quatre étages, dans lesquels ladite concentration au premier niveau pendant la durée du premier étage amène toutes les cellules du corps à entrer dans la phase de repos, et ladite concentration au deuxième niveau pendant la durée du deuxième étage permet aux cellules tumorales de reprendre leur cycle mais ne permet pas aux cellules normales de reprendre leur cycle, et ladite concentration au troisième niveau pendant la durée du troisième étage provoque la mort d'une majorité de cellules tumorales parcourant leur cycle, et ladite concentration au quatrième étage est la concentration normale qui permet aux cellules normales de reprendre leur cycle.

11. Appareil selon l'une des revendications 1 à 10, dans lequel lesdits moyens de mise en réaction comprennent un filtre sanguin extracorporel (3) et un conduit pour relier ledit filtre sanguin extracorporel (3) et ladite pompe sanguine extracorporelle (2) en une circulation extracorporelle fermée entre une artère et une veine, grâce à quoi ledit filtre sanguin extracorporel élimine sélectivement ledit acide aminé du sang.

12. Appareil selon la revendication 11, dans lequel ledit filtre (3) adsorbe sélectivement ledit acide aminé.

13. Appareil selon la revendication 11 ou 12, dans lequel ladite adsorption sélective est réalisée par une aminoacyle-ARNt synthétase spécifique audit acide aminé et reçoit son énergie de l'ATP.

14. Appareil selon la revendication 13, dans lequel ladite aminoacyle-ARNt synthéthase est liée à un gel.

15. Appareil selon l'une des revendications 11 ou 12, dans lequel ladite adsorption sélective est réalisée par un ARNt spécifique audit acide et est catalysée par une aminoacyle-ARNt synthéthase spécifique audit aminoacide et reçoit son énergie de l'ATP.

16. Appareil selon la revendication 15, dans lequel ledit ARNt est lié à un gel.

17. Appareil selon la revendication 15, dans lequel ladite aminoacyle-ARNt synthéthase est liée à un gel.

18. Appareil selon la revendication 11, dans lequel ledit filtre (3) décompose sélectivement ledit acide aminé essentiel.

19. Appareil selon la revendication 18, dans lequel ladite décomposition est réalisée par l'une des enzymes qui dégradent l'acide aminé naturel.

**20.** Appareil selon la revendication 19, dans lequel lesdites enzymes sont sélectionnées parmi les suivantes : thréonine aldolase pour la thréonine, phénylalanine hydroxylase pour la phénylalanine, arginase pour l'arginine, l'histidine ammoniac-lyase pour l'histidine, tryptophane 2,3-dioxygénase pour le tryptophane.

**21.** Appareil selon la revendication 19, dans lequel ladite enzyme est liée à un gel.

**22.** Appareil selon la revendication 11, dans lequel ledit filtre sanguin (3) pour ladite élimination sélective dudit acide aminé dudit sang incorpore un écoulement secondaire de circulation externe séparée de l'écoulement de sang par un tamis moléculaire.

**23.** Appareil selon la revendication 22, dans lequel ladite élimination sélective dudit acide aminé est réalisée par des moyens enzymatiques limités à ladite circulation secondaire.

**24.** Appareil selon l'une des revendications 11 à 23, comprenant en outre :

(a) un premier filtre extracorporel du type à fibres creuses ;
(b) une pompe extracorporelle de la fraction plasma du sang ;
(c) un deuxième filtre extracorporel du type à lit de gel ; et
(d) un conduit pour relier les composants (a), (b), (c) et ladite pompe sanguine extracorporelle de telle sorte que, en utilisation, du sang artériel est pompé par ladite pompe sanguine extracorporelle à travers lesdites fibres creuses de (a) et est renvoyé dans l'écoulement sanguin veineux, tandis que la fraction à bas poids moléculaire du plasma sanguin qui est filtré à travers les fibres creuses est pompé par (b) à travers (c) en formant une circulation secondaire externe fermée (c) et l'espace extrafibrillaire de (a), tandis que ledit premier filtre sépare les cellules sanguines et les substances à haut poids moléculaire de ladite fraction plasma du sang contenant les acides aminés, et ledit deuxième filtre extracorporel alimenté par ladite pompe de la fraction plasma du sang élimine sélectivement ledit acide aminé essentiel de ladite fraction plasma du sang.

**25.** Appareil selon la revendication 24, dans lequel ledit deuxième filtre du type à lit de gel adsorbe sélectivement ledit acide aminé.

**26.** Appareil selon la revendication 25, dans lequel ladite adsorption sélective est réalisée par une aminoacyle-ARNt synthétase spécifique audit acide aminé et reçoit son énergie de l'ATP.

**27.** Appareil selon la revendication 25, dans lequel ladite adsorption sélective est réalisée par un ARNt spécifique audit acide aminé et est catalysée par une aminoacyle-ARNt synthétase spécifique audit acide aminé et reçoit son énergie de l'ATP, et dans lequel ledit ARNt ou ladite aminoacyle-ARNt synthétase spécifiques audit acide aminé sont liés à un gel.

**28.** Appareil selon la revendication 24, dans lequel ledit deuxième filtre du type à lit de gel décomposant sélectivement ledit acide aminé essentiel.

**29.** Appareil selon la revendication 28, dans lequel ladite décomposition est réalisée par l'une des enzymes naturelles dégradant l'acide aminé.

**30.** Appareil selon la revendication 29, dans lequel ladite enzyme est choisie parmi les suivantes: thréonine aldolase pour la thréonine, phénylalanine hydroxylase pour la phénylalanine, arginase pour l'arginine, histidine ammoniac-lyase pour l'histidine, tryptophane 2,3-dioxygénase pour le tryptophane.

**31.** Appareil selon l'une des revendications 24 à 30 comprenant en outre :

(a) une première pompe à filtrat du filtre ;
(b) un deuxième filtre de type à fibres creuses ;
(c) une deuxième pompe à filtrat de filtre ;
(d) une chambre de réacteur ; et
(e) des conduits de fluide pour relier les composants (a), (b), (c), (d) et ladite pompe sanguine extracorporelle et ledit premier filtre du type à fibres creuses de telle sorte que, en utilisation, du sang artériel est pompé par ladite pompe sanguine extracorporelle à travers ledit premier filtre du type à fibres creuses et est renvoyé dans l'écoulement sanguin veineux tandis que ledit filtrat dudit premier filtre du type à fibres creuses, qui transporte la fraction à bas poids moléculaire du plasma sanguin est pompé par (a) à travers (d) et dans lesdites fibres creuses dudit deuxième filtre (b), grâce à quoi le concentré dudit deuxième filtre (b) est mélangé avec le filtrat dudit premier filtre avant son entrée dans (d), suite à quoi ledit filtrat du deuxième filtre est pompé par (c) pour être mélangé avec ledit sang artériel pour entrer dans ledit premier filtre du type à fibres creuses, suite à quoi ledit premier filtre sépare les cellules sanguines et les substances à haut poids moléculaire de ladite fraction plasma de sang contenant les acides aminés, et ledit deuxième filtre sépare les enzymes qui éliminent sélectivement au moins l'un desdits acides aminés d'un fluide de dilution du sang qui est mis en circulation entre lesdits deux filtres.

**32.** Appareil selon la revendication 31, dans lequel l'écoulement de fluide en circulation à travers les jaquettes desdits filtres est commandé par des pompes supplémentaires qui relient l'orifice d'entrée et l'orifice de sortie desdites jaquettes.

**33.** Appareil selon les revendications 31 ou 32, dans lequel lesdits filtres comprennent des moyens pour commander la pression moyenne à l'intérieur desdites jaquettes desdits filtres.

**Patentansprüche**

**1.** Vorrichtung zur Blutbehandlung, mit Mittel (2) zur Förderung des Bluts von der Eingangsöffnung (1) bis zu der Ausgangsöffnung (4) der Vorrichtung, und mit Mittel (74) zur Messung der Konzentration einer essentielen Aminosäure im Blut, gekennzeichnet durch

A) Mittel (3) zur Reaktion den niedrigmolekularen Bestandteilen des Bluts mit einem chemischen Mittel, das die Konzentration mindestens einer den essentielen Aminosäuren Arginin, Threonin, Methionin, Lysin, Valin, Leukin, Isoleukin, Histidin, Phenylalanin und Tryptophan im Blut herabstezt,
B) Mittel (70) zur Erhöhung dieser Konzentration mittels einer Zugabe dieser mindestens einer essentielen Aminosäure im Blut, und
C) Mittel (76) zur Durchführung einer vorprogrammierten Sequenz von Änderungen der Konzentration.

**2.** Vorrichtung gemäß Anspruch 1, in der die Mittel (2) zur Förderung des Bluts mindestens eine extrakorporelle Blutpumpe, vorzugsweise des peristaltischen Typs, sowie entsprechende Blutleitungen aufweisen.

**3.** Vorrichtung gemäß Anspruch 1 oder 2, in der die Mittel zur Reaktion den niedrigmolekularen Bestandteilen des Bluts mit dem chemischen Mittel mindestens einen mit einem molekularen Sieb ausgestatteten Filter aufweisen, der das chemische Mittel vom Blut trennt, wobei die niedrigmolekularen Bestandteile des Bluts, insbesondere die Aminosäuren, den Filter durchströmen und mit dem chemischen Mittel reagieren können.

**4.** Vorrichtung gemäß einem den Ansprüchen 1 bis 3, in der das chemische Mittel fähig ist, die Konzentration durch selektiven Adsorption mindestens einer den essentielen Aminosäuren abzusetzen.

**5.** Vorrichtung gemäß einem den Ansprüchen 1 bis 3, in der das chemische Mittel ein Enzym ist, das fähig ist, die Konzentration durch selektiven Zersetzung von der essentielen Aminosäuren abzusetzen.

**6.** Vorrichtung gemäß einem den Ansprüchen 1 bis 5, in der die Mittel (70) zur Erhöhung der Konzentration mittels einer Zugabe von essentielen Aminosäuren im Blut eine Injektionspumpe aufweisen.

**7.** Vorrichtung gemäß einem den Ansprüchen 1 bis 6, in der die Mittel (74) zur Messung der Konzentration der essentielen Aminosäuren im Blut eine Aminosäuren-Untersuchungsvorrichtung aufweisen.

**8.** Vorrichtung gemäß einem den Ansprüchen 1 bis 6, in der die Mittel (76) zur Durchführung einer vorprogrammierten Sequenz zur Änderung der Konzentration mindestens einen Computer aufweisen, der Angaben von den Mitteln zur Messung der Konzentration der essentielen Aminosäuren steuert und erhält, der die Mittel zur Förderung des Bluts durch die Mittel zur Reaktion den niedrigmolekularen Bestandteilen des Bluts mit chemischen Mitteln steuert, und der die Mittel zur Erhöhung der Konzentration der essentielen Aminosäuren steuert.

**9.** Vorrichtung gemäß einem den Ansprüchen 1 bis 8, in der die vorprogrammierte Sequenz sich innerhalb einer begrenzten Dauer von ein bis sieben Tage erstreckt.

**10.** Vorrichtung gemäß einem den Ansprüchen 1 bis 9, in der die vorprogrammierte Sequenz mindestens vier Stufe aufweist, wobei die während der Dauer der ersten Stufe auf dem ersten Niveau liegende Konzentration alle Zellen des Körpers in der Ruhephase einbringt, die während der Dauer der zweiten Stufe auf dem zweiten Niveau liegende Konzentration den tumoralen Zellen erlaubt, ihren Zyklus wieder zu holen, doch den normalen Zellen nicht erlaubt, ihren Zyklus wieder zu holen, und die während der Dauer der dritten Stufe auf dem dritten Niveau liegende Konzentration den Tod einer Mehrheit zyklierenden tumoralen Zellen hervorruft, wobei die Konzentration in der vierten Stufe die normale Konzentration ist, die den normalen Zellen erlaubt, ihren Zyklus wieder zu holen.

**11.** Vorrichtung gemäß einem den Ansprüchen 1 bis 10, in der die Mittel zur Reaktion einen extrakorporellen Blutfilter (3) und eine Leitung zur Verbindung des Blutfilters (3) mit der extrakorporellen Blutpumpe (2) in einem geschlossen extrakorporellen Kreislauf zwischen einer Arterie und einer Vene aufweist, wobei der Blutfilter die Aminosäure selektiv vom Blut abtrennt.

**12.** Vorrichtung gemäß Anspruch 11, in der der Filter (3) die Aminosäure selektiv adsorbiert.

**13.** Vorrichtung gemäß Anspruch 11 oder 12, in der die selektive Adsorption durch ein Aminoacyl-tRNA Synthetase erreicht wird, das für die Aminosäure spezifisch ist und seine Energie von ATP erhält.

**14.** Vorrichtung gemäß Anspruch 13, in der das Aminoacyl-tRNA Synthetase mit einem Gel fest verbunden ist.

**15.** Vorrichtung gemäß einem den Ansprüchen 11 oder 12, in der die selektive Adsorption durch eine tRNA erreicht wird, die für die Aminosäure spezifisch ist, unter Katalyse von einem Aminoacyl-tRNA Synthetase, das für die Aminosäure spezifisch ist und seine Energie von ATP erhält.

**16.** Vorrichtung gemäß Anspruch 15, in der die tRNA mit einem Gel fest verbunden ist.

**17.** Vorrichtung gemäß Anspruch 15, in der das Aminoacyl-tRNA-Synthetase mit einem Gel fest verbunden ist.

**18.** Vorrichtung gemäß Anspruch 11, in der der Filter (3) die essentiele Aminosäure selektiv zersetzt.

**19.** Vorrichtung gemäß Anspruch 18, in der die Zersetzung durch ein der Enzymen verwiklicht wird, die die natürliche Aminosäure zersetzen.

**20.** Vorrichtung gemäß Anspruch 19, in der die Enzyme zwischen den folgenden ausgewält werden: Threonin Aldolase für das Threonin, Phenylalanin Hydroxylase für das Phenylalanin, Arginase für das Arginin, Histidin Ammonia-Lyase für das Histidin, Tryptophan 2,3-Dioxygenase für das Tryptophan.

**21.** Vorrichtung gemäß Anspruch 19, in der das Enzym mit einem Gel fest verbunden ist.

**22.** Vorrichtung gemäß Anspruch 11, in der der Blutfilter (3) zur selektiven Entfernung der Aminosäure einen sekundären, externen Kreislauf aufweist, der durch ein molekulares Sieb von der Blutströmung getrennt ist.

**23.** Vorrichtung gemäß Anspruch 22, in der die selektive Ausscheidung der Aminosäure durch innerhalb des sekundären Kreislaufs begrenzte enzymatische Mittel bewirkt wird.

**24.** Vorrichtung gemäß einem den Ansprüchen 11 bis 23, die zudem die folgenden Teilen aufweist:

(a) einen ersten extrakorporellen Filter des Typs mit hohlen Fasern;
(b) eine extrakorporelle Pumpe für die Plasma-Fraktion des Bluts;
(c) einen zweiten extrakorporellen Filter des Typs mit einem Gelbett; und
(d) eine Leitung zur Verbindung der Teilen (a), (b), (c) mit der extrakorporellen Pumpe, so dass, im Betrieb, arteriellen Blut mit Hilfe der extrakorporellen Blutpumpe durch die hohlen Faser von (a) hindurchgepumpt und in die venöse Blutströmung gefordert wird, wobei die durch die hohlen Faser filtrierte niedrigmolekulare Fraktion des Blutplasmas mit Hilfe von (b) durch (c) hindurchgepumpt wird unter Ausbildung eines sekundären, externen, geschlossen Kreislaufs zwischen (c) und des extrafibrillären Raumes von (a), wobei der erste Filter die Blutzellen und die hochmolekularen Substanzen von der die Aminosäuren enthältenden Plasma-Fraktion des Bluts abtrennt, und wobei der zweite, durch die Pumpe für die Plasma-Fraktion des Bluts versorgte extrakorporelle Filter die essentiele Aminosäure von der Plasma-Fraktion des Bluts selektiv ausscheidet.

**25.** Vorrichtung gemäß Anspruch 24, in der der zweite Filter des Typs mit einem Gelbett die Aminosäure selektiv adsorbiert.

**26.** Vorrichtung gemäß Anspruch 25, in der die selektive Adsorption durch ein Aminoacyl-tRNA Synthetase bewirkt wird, das für die Aminosäure spezifisch ist und seine Energie von ATP erhält.

**27.** Vorrichtung gemäß Anspruch 25, in der die selektive Adsorption durch eine tRNA bewirkt wird, die für die Aminosäure spezifisch ist, unter Katalyse von einem Aminoacyl-tRNA Synthetase, das für die Aminosäure spezifisch ist und seine Energie von ATP erhält, und die tRNA oder das Aminoacyl-tRNA Synthetase, die für die Aminosäure spezifisch sind, mit einem Gel fest verbunden sind.

**28.** Vorrichtung gemäß Anspruch 24, in der der zweite Filter des Typs mit einem Gelbett selektiv die essentiele Aminosäure zersetzt.

**29.** Vorrichtung gemäß Anspruch 28, in der die Zersetzung durch ein den die Aminosäure zersetzenden, natürlichen Enzymen bewirkt wird.

**30.** Vorrichtung gemäß Anspruch 29, in der die Enzyme zwischen den folgenden ausgewählt werden: Threonin Aldolase für das Threonin, Phenylalanin Hydroxylase für das Phenylalanin, Arginase für das Arginin, Histidin Ammonia-Lyase für das Histidin, Tryptophan 2,3-Dioxygenase für das Tryptophan.

**31.** Vorrichtung gemäß einem den Ansprüchen 24 bis 30, die zudem die folgenden Teilen aufweist:

(a) eine erste Pumpe für das Filtrat vom Filter;

(b) einen zweiten Filter des Typs mit hohlen Fasern;

(c) eine zweite Pumpe für das Filtrat vom Filter;

(d) eine Reaktorkammer; und

(e) Flüssigkeitsleitungen zur Verbindung der Teilen (a), (b), (c), (d) mit der extrakorporellen Blutpumpe und dem ersten Filter des Typs mit hohlen Fasern, so dass, im Betrieb, arteriellen Blut mit Hilfe der extrakorporellen Blutpumpe durch die hohlen Faser von (a) hindurchgepumpt und in die venöse Blutströmung gefordert wird, wobei das Filtrat vom Filter des Typs mit hohlen Fasern, der die niedermolekulare Plasma-Fraktion des Bluts mit Hilfe von (a) durch (d) und die hohlen Faser des zweiten Filter (b) hindurchgepumpt wird, wobei das Konzentrat vom zweiten Filter (b) mit dem Filtrat vom ersten Filter vor dem Eintritt in (d) hinein gemischt wird, wobei das Filtrat vom zweiten Filter mit Hilfe von (c) zur Mischung mit dem arteriellen Blut und zum Eintritt in den ersten Filter des Typs mit hohlen Fasern gepumpt wird, wobei der erste Filter die Blutzellen und die hochmolekulare Substanzen von der die Aminosäuren enthältenden Plasma-Fraktion des Bluts abtrennt, und wobei der zweite Filter die mindestens eine der Aminosäuren selektiv zersetzenden Enzyme von einer zwischen den beiden Filtern zirkulierten Blutverdünnungsflüssigkeit abtrennt.

**32.** Vorrichtung gemäß Anspruch 31, in der die durch die Filtermantel zirkulierte Flüssigkeitssströmung durch zusätzliche, die Eingangsöffnung mit der Ausgangsöffnung der Filtermanteln verbindende Pumpen angetreibt wird.

**33.** Vorrichtung gemäß Anspruch 31 oder 32, in der die Filter Mittel zur Steuerung des durchschnittlichen Drucks innerhalb der Filtermanteln aufweisen.

FIGURE 1

FIGURE 2

FIGURE 3

FIGURE 4

FIGURE 5

FIGURE 6

FIGURE 7

EP 0 671 958 B1

EP 0 671 958 B1

FIGURE 8

26

FIGURE 9

15    16    5

12

FIGURE    10

17    15    16    18    19

12

FIGURE    11

FIGURE 12

FIGURE 13

FIGURE 14

EP 0 671 958 B1

FIGURE 15

FIGURE 16

EP 0 671 958 B1

32